# EUROPEAN PATENT APPLICATION

(11) **EP 4 852 015 A1**
(43) Date of publication of application: **09.12.2026**
(21) Application number: 25190653.3
(22) Date of filing: 21.07.2025
(51) Int. Cl.: G16H 40/63, G16H 20/40, A61B 18/00

(54) **HANDHELD ELECTROTHERMAL THERAPY DEVICE AND ABLATION PLANNING METHOD**

(30) Priority: 05.06.2025 CN 202510750655
(71) Applicant: Canyon Medical Inc., Nanjing, Jiangsu 210032 (CN)
(72) Inventor: LI, Ran, Nanjing, 210032 (CN); PAN, Hui, Nanjing, 210032 (CN); LONG, Long, Nanjing, 210032 (CN); LIU, Kunlun, Nanjing, 210032 (CN)
(74) Representative: Doherty, William

(57) **Abstract**

The present disclosure belongs to the technical field of tumor ablation planning, and specifically relates to a handheld electrothermal therapy device and an ablation planning method. **In** the present disclosure, through the accurate prediction on the battery attenuation trend and the dynamic adjustment on the ablation strategy, it significantly improves the efficiency and safety of the ablation treatment; through the multi-dimensional screening and verification of the candidate attenuation curve, it ensures the accuracy of the target attenuation curve and lays a foundation for the subsequent steps; through the self-adaptive adjustment mechanism of the sliding evaluation window, it makes the evaluation process more flexible and efficient, which can accurately capture minor changes in the battery attenuation; at the same time, through providing the correction strategy for the target attenuation curve, it further enhances the accuracy of the prediction and ensures an accurate estimation for the battery remaining usage duration; and finally, through the dynamic adjustment on the ablation segment length and the current output based on the remaining usage duration, it not only optimizes the ablation effect, but also effectively prolongs the service life of the device.

## Description

### TECHNICAL FIELD

The present disclosure belongs to the technical field of tumor ablation planning, and specifically relates to a handheld electrothermal therapy device and an ablation planning method.

### BACKGROUND ART

Currently, in clinical practice, after performing a needle biopsy in a solid tumor of a liver, a kidney, a lung, or other areas, the hemostasis or cauterization is generally not performed on the interior of the solid tumor or on the puncture needle tract. Internal bleeding of the solid tumor may increase the risk of infection, which further aggravates the condition of the patient and results in delayed treatment. The internal bleeding of the solid tumor may lead to the hematomas, which compress surrounding tissues and organs, thereby causing pain and functional disorders. Due to the higher risk of bleeding at certain biopsy sites or coagulation disorders of patients, doctors often choose not to perform a biopsy or to use a finer needle for the biopsy, which may result in inadequate preoperative biopsy pathological assessment or lower biopsy tissue effectiveness, thereby affecting the determination of the clinical treatment plan. For biopsies of malignant tumors, if the puncture needle tract is not treated, there is a certain probability that tumor cells may implant in the puncture needle tract, which results in the metastasis and spread of tumor cells and further aggravates the condition of the patient. Most of the current ablation devices are connected to the main unit by connecting wires. The operator is often disturbed by the connecting wires during operations, which makes the operation very inconvenient. Some heavy connecting wires even require external brackets to ensure the normal operation of the ablation procedure, which undoubtedly increases the complexity of the ablation surgery. Based on this, the solution proposes a handheld electrothermal therapy device and an ablation planning method to address the above problems.

### SUMMARY

The objective of the present disclosure is to provide a handheld electrothermal therapy device and an ablation planning method, which can dynamically adjust an ablation strategy according to a real-time battery status, so as to ensure the safety and effectiveness of the ablation process.

The technical solutions adopted by the present disclosure are specified as follows.

An ablation planning method is provided, including:
constructing a reference attenuation curve database based on an output voltage, an output current, and a battery capacity in a historical database;
collecting an initial voltage, an initial current, an initial capacity of a battery under a load condition of a constant output voltage, collecting a real-time voltage, a real-time current, and a remaining capacity of the battery according to a preset time interval, and fitting a real-time attenuation curve;
comparing the real-time attenuation curve with reference attenuation curves in a reference attenuation curve database, selecting a reference attenuation curve whose matching degree exceeds a preset matching threshold, summarizing it into a candidate attenuation curve dataset, and determining a target attenuation curve adapted to the real-time voltage according to the candidate attenuation curve dataset;
intercepting a sliding evaluation window according to a deviation between the target attenuation curve and real-time attenuation curve starting from a current ablation node within a remaining usage duration, and correcting the target attenuation curve according to an actual attenuation trend of the battery within the sliding evaluation time window; and
predicting a remaining usage duration of the battery according to the corrected target attenuation curve, and dynamically adjusting an ablation segment length of an ablation needle according to the remaining usage duration.

In a preferred solution, the historical database includes a historical output voltage, a historical output current, and a historical battery capacity under a multi-environmental condition, and the multi-environmental condition includes temperature, humidity, and load fluctuation, wherein
when constructing the reference attenuation curve according to the historical output voltage, the historical output current, and the historical battery capacity, the specific process includes:
performing a pre-process on the historical output voltage, the historical output current, and the historical battery capacity to remove abnormal data, and performing normalization processing to unify to the same order of magnitude;
performing a cluster analysis on the historical output voltage, the historical output current, and the historical battery capacity according to the multi-environmental condition, so as to obtain a historical data subset under each condition; and
fitting the historical output voltage, the historical output current, and the historical battery capacity in each historical data subset to obtain the reference attenuation curve under each condition, and storing the reference attenuation curve into the reference attenuation curve database.

In a preferred solution, the step of collecting a real-time voltage, a real-time current, and a remaining capacity of the battery according to a preset time interval, and fitting a real-time attenuation curve includes:
dynamically adjusting a sampling interval duration based on a preset ratio according to a current operating temperature and a load fluctuation intensity of the battery;
collecting the real-time voltage and the real-time current of the battery in real time based on the dynamically adjusted sampling interval duration, and recording the remaining capacity of the battery at a corresponding moment; and
fitting the real-time voltage, the real-time current, and the remaining capacity of the battery by using a nonlinear regression method, so as to obtain the real-time attenuation curve of the battery.

In a preferred solution, the step of comparing the real-time attenuation curve with reference attenuation curves in a reference attenuation curve database, and selecting a reference attenuation curve whose matching degree exceeds a preset matching threshold includes:
assessing a morphological consistency between the real-time attenuation curve and the reference attenuation curve through a waveform comparison;
performing a time axis calibration on curves under different collection frequencies, and eliminating a sampling interval error by using difference compensation, so as to obtain the calibrated real-time attenuation curve and reference attenuation curve;
establishing a two-stage screening mechanism, wherein a reference attenuation curve with a morphological consistency higher than a preliminary threshold is retained under a first-stage screening mechanism, and an abnormal matching curve in the remaining reference attenuation curves is eliminated by a statistical validation under a second-stage screening mechanism; and
taking the remaining reference attenuation curves after screened by the two-stage screening mechanism as the candidate attenuation curves, summarizing them into the candidate attenuation curve dataset.

In a preferred solution, the step of determining a target attenuation curve adapted to the real-time voltage according to the candidate attenuation curve dataset includes:
performing time synchronization processing on the candidate attenuation curve and the real-time attenuation curve, calculating a time series similarity between the candidate attenuation curve and the real-time attenuation curve, and screening a candidate attenuation curve exceeding a time series similarity threshold;
acquiring a current environmental parameter, and executing a multi-level filtering operation according to a different value between a historical temperature and a current temperature, a load fluctuation amplitude, and an environmental humidity; and
performing a trend validation on the candidate attenuation curves passing the multi-level filtering operation, and selecting a candidate attenuation curve with the highest matching degree of the attenuation trend to the real-time attenuation trend as the target attenuation curve.

In a preferred solution, the step of intercepting a sliding evaluation window according to a deviation between the target attenuation curve and real-time attenuation curve includes:
tracing back a sample duration on the basis of a current node, and setting multiple sampling nodes within the sample duration;
obtaining a cumulative deviation value between the target attenuation curve and the real-time attenuation curve at each sampling node, and triggering a window interception mechanism when the cumulative deviation value exceeds a preset deviation threshold;
calculating a deviation change rate of the cumulative deviation value under each sampling node, and adaptively adjusting a length of the duration of the sliding evaluation window according to the deviation change rate;
extending the length of duration of the sliding evaluation window to twice a length of baseline when deviation change rates of N consecutive sampling nodes exceed a preset deviation change rate threshold;
shortening the length of duration of the sliding evaluation window to half of the length of baseline when the deviation change rates of N consecutive sampling nodes are lower than the preset deviation stability threshold;
adjusting the length of duration of the sliding evaluation window according to a cumulative value of the deviation change rates when the deviation change rates at N sampling nodes exceed the preset deviation change rate threshold and are lower than the preset deviation stability threshold simultaneously; and
proportionally increasing the length of duration of the sliding evaluation window when the cumulative value of the deviation change rates is positive, and proportionally reducing the length of duration of the sliding evaluation window when the cumulative value of the deviation change rates is negative.

In a preferred solution, the step of correcting the target attenuation curve according to an actual attenuation trend of the battery within the sliding evaluation time window includes:
extracting the real-time voltage, the real-time current, and a capacity attenuation rate of the battery in the sliding evaluation window, and eliminating high-frequency noise by sliding average filtering;
performing a residual analysis on an actual attenuation rate of the sliding evaluation window and a theoretical value of the target attenuation curve to obtain a residual distribution characteristic; and
performing piecewise linear fitting on the target attenuation curve based on the residual distribution characteristic to obtain a correction coefficient, and applying the correction coefficient to a corresponding duration of the target attenuation curve to correct the target attenuation curve, wherein
the target attenuation curve is verified by a validation mechanism after each correction; and
when a deviation value between the corrected target attenuation curve and the real-time attenuation curve exceeds a preset verification threshold range under multiple consecutive validation nodes, rolling back to a previous validated correction state, and shortening the length of duration of the sliding evaluation window.

In a preferred solution, the step of predicting a remaining usage duration of the battery according to the corrected target attenuation curve, and dynamically adjusting an ablation segment length of an ablation needle according to the remaining usage duration includes:
extracting a correlation function between the capacity attenuation rate and the output voltage based on the corrected target attenuation curve, and calculating a time point of a current capacity reaching a preset failure threshold based on the correlation function, so as to obtain the remaining usage duration of the battery;
establishing a mapping relationship between an ablation segment length and the remaining usage duration, determining the ablation segment where the ablation needle is currently located when the remaining usage duration is smaller than or equal to the preset stability threshold;
reducing the current output when the ablation segment at which the ablation needle currently is located is short-segment ablation; adjusting the long-segment ablation to medium-segment ablation when the current ablation segment is long-segment ablation; shortening an ablation segment length to the short-segment ablation when the remaining usage duration is still insufficient under the medium segment, and continuously reducing the current output; and
maintaining the current ablation segment length and current output unchanged when the remaining usage duration is larger than the preset stability threshold, and monitoring the battery in real time until the ablation process ends.

In a preferred solution, the ablation needle includes an electrocoagulation function and an ablation function when performing the ablation, wherein
the electrocoagulation function serves for hemostasis and tissue coagulation;
the ablation function serves for an ablation treatment of a target tissue; and
under both the electrocoagulation function and the ablation function, a voltage of each segment of electric heating wire is regulated according to the temperature feedback of temperature measurement components, so as to control the temperature of an entire treatment segment, wherein
the number of the temperature measurement components is larger than the number of segments of electric heating wires, and the temperature measurement components are configured to detect a temperature of a non-target treatment segment.

The present disclosure further provides a handheld electrothermal therapy device applied to the above ablation planning method, and the electrothermal therapy device includes:
a handle, wherein the handle includes a power supply unit inside, and the power supply unit is configured to provide energy for the ablation and/or electrocoagulation treatment;
a heating head, wherein the heating head is provided with a plurality of groups of filling layers, temperature measurement units, central conductors, and electric heating wires, so that the heating head performs the electrocoagulation and/or ablation treatment on target tissues of different segment lengths; and
a needle rod, wherein one end of the needle rod is connected to the handle; one end of the needle rod away from the handle is connected to the heating head; and the energy of the power supply unit is transferred to the heating head by conductors inside the needle rod; and
during the ablation and/or electrocoagulation treatment, the device can obtain a remaining usage duration of the power supply unit according to the attenuation trend of the power supply unit during use, and perform a dynamic adjustment on the ablation segment length according to the remaining usage duration.

In a preferred solution, a coaxial puncture needle for use in conjunction with the electrothermal therapy device is provided when the electrothermal therapy device is in use, wherein the electrothermal therapy device achieves the electrocoagulation and/or ablation treatment through a channel established by the coaxial puncture needle.

In a preferred solution, the electric heating wires are configured to heat the heating head, and a plurality of groups of electric heating wires are provided, wherein one end of each group of electric heating wires is connected to the power supply, and the other end is connected to the central conductor.

In a preferred solution, the central conductor is a negative electrode end of all electric heating wires, and a loop is formed between the central conductor and the electric heating wires.

In a preferred solution, each of the temperature measurement units is configured to monitor a temperature of a current position, and the number of the temperature measurement units is larger than the number of the electric heating wires.

In a preferred solution, the electrocoagulation function serves for hemostasis and coagulation of the tissue;
the ablation function serves for the ablation treatment of the tissue;
under both the electrocoagulation function and the ablation function, the voltage of each segment of electric heating wire is regulated according to the temperature feedback of temperature measurement units, so as to control the temperature and the duration of the entire treatment segment, thereby realizing the electrocoagulation function and the ablation function.

The beneficial effects of the present disclosure are as follows.

In the present disclosure, through the accurate prediction on the battery attenuation trend and the dynamic adjustment on the ablation strategy, it significantly improves the efficiency and safety of the ablation treatment; through the multi-dimensional screening and verification of the candidate attenuation curve, it ensures the accuracy of the target attenuation curve and lays a foundation for the subsequent steps; the self-adaptive adjustment mechanism of the sliding evaluation window makes the evaluation process more flexible and efficient, which can accurately capture minor changes in the battery attenuation; at the same time, the provision of the correction strategy for the target attenuation curve further enhances the accuracy of the prediction and ensures an accurate estimation for the battery remaining usage duration; and finally, through the dynamic adjustment on the ablation segment length and the current output based on the remaining usage duration, it not only optimizes the ablation effect, but also effectively prolongs the service life of the device.

### BRIEF DESCRIPTION OF DRAWINGS

FIG. 1 shows a schematic flow diagram of a method of the present disclosure;
FIG. 2 shows a structure schematic diagram of a device of the present disclosure;
FIG. 3 shows an internal schematic diagram of a heating head of the present disclosure; and
FIG. 4 shows a circuit structure schematic diagram of the present disclosure.

In the drawings, the components represented by reference numbers are listed below:
1, handle; 2, heating head; 3, needle rod; 4, coaxial puncture needle;
101, filling layer; 102, temperature measurement unit; 103, central conductor; and 104, electric heating wire.

### DETAILED DESCRIPTION OF EMBODIMENTS

In order to make the above objectives, features, and advantages of the present disclosure more obvious and understandable, the specific embodiments of the present disclosure are described in detail below in conjunction with the specification and drawings.

Many specific details in the following description are provided to facilitate a full understanding of the present disclosure, but the present disclosure can be implemented in other ways different from those described herein. A person skilled in the art can make similar generalizations without violating the connotations of the present disclosure, and thus the present disclosure is not limited by the specific embodiments disclosed below.

Next, the term "an embodiment" or "embodiment" as used herein refers to a specific feature, structure, or characteristic that can be contained in at least one embodiment of the present disclosure. The phrase "in a preferred embodiment" that appears in different places in the specification does not always refer to the same embodiment, nor is it a separate or selective embodiment that is mutually exclusive with other embodiments.

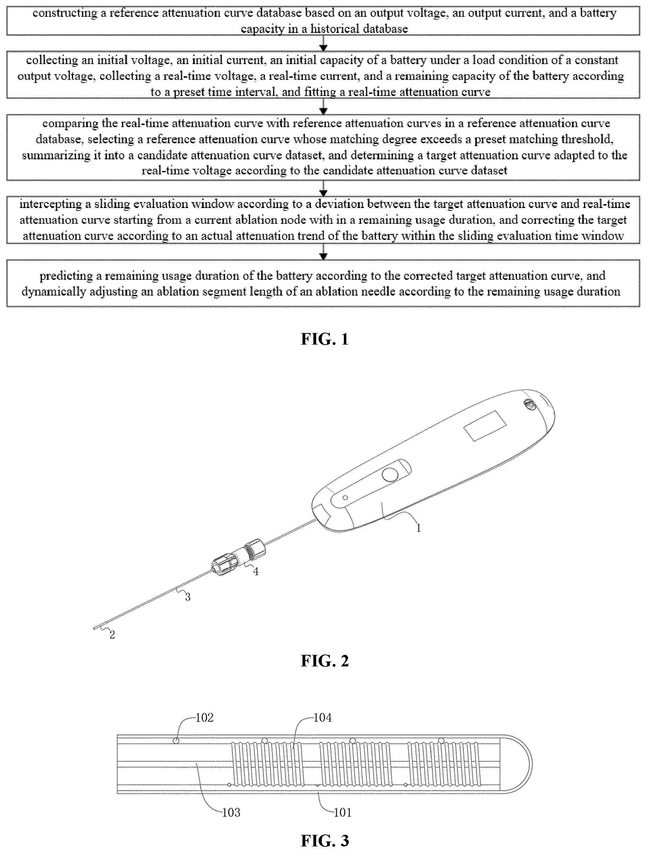

Referring to FIG. 1, the present disclosure provides an ablation planning method, including steps S1~S5.

S1: constructing a reference attenuation curve database based on an output voltage, an output current, and a battery capacity in a historical database.

In the step S1, when performing the ablation operation on the solid tumor, the handheld electrothermal therapy device can adjust the temperature of the heating zone to complete the needle coagulation or ablation. When the handheld electrothermal therapy device is in operation, it will monitor and record an output voltage, output current, and battery capacity during its historical working process, and summarize them into a historical database. Based on data in the historical database, a reference attenuation curve of the battery of the handheld electrothermal therapy device can be constructed, so as to determine typical attenuation modes of the battery in different conditions. The historical database includes a historical output voltage, a historical output current, and a historical battery capacity under a multi-environmental condition, and the multi-environmental condition includes temperature, humidity, and load fluctuation.

When constructing the reference attenuation curve according to the historical output voltage, the historical output current, and the historical battery capacity, the specific process includes:
performing a pre-process on the historical output voltage, the historical output current, and the historical battery capacity to remove abnormal data, and performing normalization processing to unify to the same order of magnitude;
performing a cluster analysis on the historical output voltage, the historical output current, and the historical battery capacity according to the multi-environmental condition, so as to obtain a historical data subset under each condition; and
fitting the historical output voltage, the historical output current, and the historical battery capacity in each historical data subset to obtain the reference attenuation curve under each condition, and storing it into the reference attenuation curve database.

Specifically, the historical database includes the records of the battery performances under multiple environmental conditions. The multi-environmental condition includes the temperature fluctuation, the humidity change, and the load instability, all of which have a significant impact on the output voltage, output current, and capacity of the battery. Therefore, the analysis and effective utilization of historical data serve as a reference foundation for assessing the battery performance attenuation trend. During the process of constructing the reference attenuation curve, it needs to perform the data pre-processing first. During this stage, the abnormal data points caused by the measurement error or the abnormal event will be eliminated to ensure the accuracy and reliability of subsequent analysis. Then, it is to perform the normalization processing on the remaining normal data to adjust all measurement values to the same order of magnitude, which facilitates the cross-condition comparison and analysis. Subsequently, the historical data is placed within a framework of multi-environmental condition for cluster analysis. By using clustering algorithms such as K-means clustering, the historical data can be divided into multiple historical data subsets with similar environmental characteristics. Each historical data subset represents a battery operation mode under a certain condition, so as to reveal the relationship between the environmental factor and the battery performance. When independently analyzing each data subset, the historical output voltage, historical output current, and historical battery capacity are fitted respectively. This process can be based on linear or non-linear fitting methods, such as the least squares method or Gaussian process regression, so as to construct the reference attenuation curve that best describes the data trend. The reference attenuation curve is mainly used to depict the attenuation process of battery under various conditions, thereby providing a pre-reference basis for predicting the battery performance under similar conditions in the future. The reference attenuation curve obtained by fitting will be stored in a pre-constructed reference attenuation curve database, and subsequently, the corresponding reference attenuation curve can be quickly retrieved and applied to improve the efficiency of subsequent battery analysis.

S2: collecting an initial voltage, an initial current, an initial capacity of a battery under a load condition of a constant output voltage, collecting a real-time voltage, a real-time current, and a remaining capacity of the battery according to a preset time interval, and fitting a real-time attenuation curve.

In the step S2, when the battery operates under a constant load, the system will collect the initial voltage, the initial current, and the initial capacity of the battery in real time, and as time passes, the real-time voltage, the real-time current, and the remaining capacity of the battery will be continuously monitored and recorded based on the preset time interval. Based on this, a real-time attenuation curve reflecting a current attenuation state of the battery can be fitted. The step of collecting a real-time voltage, a real-time current, and a remaining capacity of the battery according to a preset time interval, and fitting a real-time attenuation curve includes:
dynamically adjusting a sampling interval duration based on a preset ratio according to a current operating temperature and a load fluctuation intensity of the battery;
collecting the real-time voltage and the real-time current of the battery in real time based on the dynamically adjusted sampling interval duration, and recording the remaining capacity of the battery at a corresponding moment; and
fitting the real-time voltage, the real-time current, and the remaining capacity of the battery by using a nonlinear regression method, so as to obtain the real-time attenuation curve of the battery.

Specifically, during the fitting process of the real-time attenuation curve, considering that battery performance may be affected by various factors such as operating temperature and load fluctuation intensity, the embodiment adopts a method of dynamically adjusting the sampling interval duration when collecting real-time data, so as to adapt to different conditions. Specifically, the sampling interval duration will be dynamically adjusted based on a preset ratio according to a current operating temperature and a load fluctuation intensity of the battery. When the condition is relatively stable, the sampling interval can be appropriately extended to reduce the data redundancy and improve the processing efficiency. When the condition fluctuates significantly, the sampling interval is shortened to capture more data details and to ensure the accuracy of the real-time attenuation curve. After determining the sampling interval, the real-time voltage and the real-time current of the battery will be collected in real time based on this dynamically adjusted sampling interval duration, and the remaining capacity of the battery at the corresponding moment is recorded. Finally, the collected real-time voltage, the real-time current, and the remaining capacity of the battery are fitted by using a nonlinear regression method, so as to obtain the real-time attenuation curve of the battery. The real-time attenuation curve reflects the current attenuation state of the battery, and serves as the primary basis for assessing the remaining life and performance attenuation trend of the battery. The nonlinear regression method can be the local weighted regression, support vector regression, neural network regression, etc., all of which can capture nonlinear relationships in the data, thereby improving the accuracy and reliability of fitting. Through the real-time attenuation curve, the performance change including the voltage fluctuation, current consumption, and capacity attenuation trend of the battery under the constant load condition can be intuitively observed, so as to adjust the treatment parameters in a timely manner during the ablation process and ensure the safety of the use of electrothermal therapy device.

S3: comparing the real-time attenuation curve with reference attenuation curves in a reference attenuation curve database, selecting a reference attenuation curve whose matching degree exceeds a preset matching threshold, summarizing it into a candidate attenuation curve dataset, and determining a target attenuation curve adapted to the real-time voltage according to the candidate attenuation curve dataset.

In step S3, after the real-time attenuation curve is output, it will be compared with numerous reference attenuation curves stored in the reference attenuation curve database. This process specifically involves the calculation of curve similarity, such as quantifying the matching degree by calculating indicators including the Euclidean distance, Manhattan distance, or cosine similarity between two curves. Then, the reference attenuation curves with higher matching degrees will be screened out according to a preset matching threshold, and the screened reference attenuation curves will form the candidate attenuation curve dataset. The candidate attenuation curve dataset provides multiple possible reference benchmarks for evaluating the current battery performance. Finally, the correlation between the candidate attenuation curve dataset and the real-time attenuation curve will be further analyzed to determine the target attenuation curve that is most adaptive to the real-time voltage. The step of comparing the real-time attenuation curve with reference attenuation curves in a reference attenuation curve database, and selecting a reference attenuation curve whose matching degree exceeds a preset matching threshold includes:
assessing a morphological consistency between the real-time attenuation curve and the reference attenuation curve through a waveform comparison;
performing a time axis calibration on curves under different collection frequencies, and eliminating a sampling interval error by using difference compensation, so as to obtain the calibrated real-time attenuation curve and reference attenuation curve;
establishing a two-stage screening mechanism, wherein a reference attenuation curve with a morphological consistency higher than a preliminary threshold is retained under a first-stage screening mechanism, and an abnormal matching curve in the remaining reference attenuation curves is eliminated by a statistical validation under a second-stage screening mechanism; and
taking the remaining reference attenuation curves after screened by the two-stage screening mechanism as the candidate attenuation curves, summarizing them into the candidate attenuation curve dataset.

Specifically, when initially screening reference attenuation curves, it will perform the waveform comparison to visually assess the morphological consistency between the real-time attenuation curve and each reference attenuation curve, where the morphological consistency reflects the consistency of the attenuation trend between the reference attenuation curve and the real-time attenuation curve. Considering that there may be misalignment on the time axis due to the difference in sampling frequencies when collecting the real-time attenuation curve and the reference attenuation curve, the time axis calibration processing will be performed before comparison, and the difference compensation is used to eliminate sampling interval errors, which ensures the consistency between the reference attenuation curve and the real-time attenuation curves on the time axis, so as to obtain the calibrated real-time attenuation curve and reference attenuation curve and improve the accuracy of the comparison result. Next, in order to screen out the candidate curve that best matches the real-time attenuation curve from the numerous reference attenuation curves, the embodiment establishes a two-stage screening mechanism. Under the first-stage screening mechanism, it presets a preliminary threshold, and retains the reference attenuation curves with the morphological consistency higher than this preliminary threshold as a preliminary screening result, which can quickly eliminate those reference curves that are significantly inconsistent with the real-time attenuation curve morphology, narrow the candidate range, and improve the efficiency of subsequent screening. After entering the second-stage screening mechanism, it will adopt the statistical validation method to further validate the remaining reference attenuation curves so as to eliminate abnormal matching curves, which ensures that the final selected candidate attenuation curve dataset has higher accuracy and reliability. For example, by calculating statistical indices such as the correlation coefficient and the standard deviation between the remaining reference attenuation curve and the real-time attenuation curves, the matching degree can be quantified, and those abnormal matching curves with statistical indices significantly deviating from the normal range can be eliminated. Finally, it takes the remaining reference attenuation curves after screened by the two-stage screening mechanism as the candidate attenuation curves, and summarizes them into the candidate attenuation curve dataset, thereby providing a reference for determining the target attenuation curves adapted to the real-time voltage in subsequent steps.

Then, the step of determining a target attenuation curve adapted to the real-time voltage according to the candidate attenuation curve dataset includes:
performing time synchronization processing on the candidate attenuation curve and the real-time attenuation curve, calculating a time series similarity between the candidate attenuation curve and the real-time attenuation curve, and screening a candidate attenuation curve exceeding a time series similarity threshold;
acquiring a current environmental parameter, and executing a multi-level filtering operation according to a different value between a historical temperature and a current temperature, a load fluctuation amplitude, and an environmental humidity; and
performing a trend validation on the candidate attenuation curve passing the multi-level filtering operation, and selecting the candidate attenuation curve with the highest matching degree of the attenuation trend to the real-time attenuation trend as the target attenuation curve.

In the embodiment, after the candidate attenuation curve dataset is determined, it will perform the time synchronization processing on the candidate attenuation curve and the real-time attenuation curve, so as to ensure that the candidate attenuation curve and the real-time attenuation curve are comparable in the time dimension. The time synchronization processing can eliminate the error due to the time misalignment, so that the candidate attenuation curve and the real-time attenuation curve can be aligned at the same time point. Next, a time series similarity between the candidate attenuation curve and the real-time attenuation curve is calculated, wherein the time series similarity is an index for measuring the similarity degree of two curves in the time series, and the matching degree between the candidate attenuation curve and the real-time attenuation curve can be quantified by calculating the time series similarity. The formula for calculating the time series similarity is: time series similarity $score = 1 - \frac{{\sum }_{i = 1}^{N} \left|{P}_{i}-{Q}_{i}\right|}{N ⋅ max \left(\left|{P}_{i}\right|, \left|{Q}_{i}\right|\right)}$, where *Pᵢ* represents a value of the real-time attenuation curve at *i*-th time point on the time series; *Qᵢ* represent a value of the candidate attenuation curve at *i*-th time point on the time series; and *N* represent a total number of the time points on the time series. The higher the time series similarity score is, the higher the similarity degree between the candidate attenuation curve and the real-time attenuation curve on the time series. By presetting the time series similarity threshold, the candidate attenuation curve that is highly similar to the real-time attenuation curve on the time series can be screened out. Subsequently, the current environmental parameter will be acquired, where the environmental parameter includes, but is not limited to the current temperature, historical temperature, load fluctuation amplitude, and environmental humidity, etc., and changes in the environmental parameter may have an effect on the attenuation trend of the battery. Therefore, when determining the target attenuation curve, the multi-level filtering operation will be executed according to parameters such as the different value between the current temperature and historical temperature, load fluctuation amplitude, and environmental humidity. The multi-level filtering operation can further eliminate those candidate attenuation curves that are significantly influenced by the environmental factors and are not consistent with the current actual condition, thereby improving the accuracy and reliability of the final determined target attenuation curve. Finally, a trend validation is performed on the candidate attenuation curve passing the multi-level filtering operation. Specifically, it is to assess the matching degree between the attenuation trend of the candidate attenuation curve and the real-time attenuation trend by analyzing the attenuation trend of the candidate attenuation curve. During the process, the indices such as similarity or difference in the attenuation trend between the candidate attenuation curve and the real-time attenuation curve are calculated, and the candidate attenuation curve with the highest matching degree of the attenuation trend to the real-time attenuation trend is selected as the target attenuation curve based on this, thereby providing a more precise voltage adaptation reference for the electrothermal therapy device and ensuring the safety and effectiveness of the electrothermal therapy device during the ablation process.

S4: intercepting a sliding evaluation window according to a deviation between the target attenuation curve and real-time attenuation curve starting from a current ablation node within a remaining usage duration, and correcting the target attenuation curve according to an actual attenuation trend of the battery within the sliding evaluation time window.

In step S4, during the tumor ablation, in order to ensure a stable output of the electrothermal therapy device throughout the entire treatment process, it needs to constantly correct the target attenuation curve based on the real-time attenuation curve. First, it will dynamically intercept a sliding evaluation window according to a deviation condition between the target attenuation curve and real-time attenuation curve starting from the current ablation node within the remaining usage duration. The size of the sliding evaluation window can be adjusted according to the actual situation, so as to ensure that it covers enough data points, thereby more accurately reflecting the attenuation trend of the battery. In the sliding evaluation time window, the actual battery attenuation will be monitored in real time and compared with the target attenuation curve. If a larger deviation is found between the two, the target attenuation curve will be corrected accordingly to ensure that it is always consistent with the actual attenuation condition of the electrothermal therapy device, thereby providing a more precise voltage adaptation reference for the electrothermal therapy device. The step of intercepting a sliding evaluation window according to a deviation between the target attenuation curve and the real-time attenuation curve includes:
tracing back a sample duration on the basis of a current node, and setting multiple sampling nodes within the sample duration;
obtaining a cumulative deviation value between the target attenuation curve and the real-time attenuation curve at each sampling node, and triggering a window interception mechanism when the cumulative deviation value exceeds a preset deviation threshold;
calculating a deviation change rate of the cumulative deviation value under each sampling node, and adaptively adjusting a length of duration of the sliding evaluation window according to the deviation change rate;
extending the length of duration of the sliding evaluation window to twice a length of baseline when deviation change rates of N consecutive sampling nodes exceed a preset deviation change rate threshold;
shortening the length of duration of the sliding evaluation window to half of the length of baseline when the deviation change rates of N consecutive sampling nodes are lower than the preset deviation stability threshold;
adjusting the length of duration of the sliding evaluation window according to a cumulative value of the deviation change rates when the deviation change rates at N sampling nodes exceed the preset deviation change rate threshold and are lower than the preset deviation stability threshold simultaneously, wherein
proportionally increasing the length of duration of the sliding evaluation window when the cumulative value of the deviation change rates is positive, and proportionally reducing the length of duration of the sliding evaluation window when the cumulative value of the deviation change rates is negative.

Specifically, when intercepting the sliding evaluation window, the current ablation treatment node is first taken as the time reference point, and then a corresponding sample duration is traced back towards the past. In the sample duration, multiple sampling nodes will be evenly set to facilitate the comparative and analysis of the target attenuation curve and the real-time attenuation curve. The cumulative deviation value between the target attenuation curve and the real-time attenuation curve will be calculated on each sampling node, and the cumulative deviation value reflects the overall difference condition between the target attenuation curve and the real-time attenuation curve in the sample duration. When the cumulative deviation value exceeds the preset deviation threshold, it indicates that the difference between the target attenuation curve and the real-time attenuation curve is large enough, and a window interception mechanism needs to be triggered for further evaluation. Then, it will calculate the deviation change rate of the cumulative deviation value at each sampling node, and the deviation change rate reflects the change trend of the cumulative deviation value over time. Next, it is to adaptively adjust the length of duration of the sliding evaluation window according to the magnitude of the deviation change rate. If the deviation change rates at N consecutive sampling nodes all exceed the preset deviation change rate threshold (where the value of N is not less than 3), it indicates that the difference between the target attenuation curve and the real-time attenuation curve is rapidly expanding. At this time, the length of duration of the sliding evaluation window needs to be extended to twice the length of baseline, so as to cover more data points to more accurately reflect the attenuation trend of the battery. Conversely, if the deviation change rates at N consecutive sampling nodes are all lower than the preset deviation stability threshold, it indicates that the difference between the target attenuation curve and the real-time attenuation curve tends to be stabilized. At this time, the length of baseline of the sliding evaluation window can be reduced to half of the length of baseline to improve the efficiency of evaluation. In the practical applications, the situation of which the deviation change rates at N sampling nodes exceed the preset deviation change rate threshold and are lower than the preset deviation stability threshold simultaneously exists. At this time, the length of duration of the sliding evaluation window needs to be adjusted according to the cumulative value of the deviation change rates. If the cumulative value of the deviation change rate is positive, it means that the difference between the target attenuation curve and the real-time attenuation curve is still expanding, at which time the length of duration of the sliding evaluation window needs to be increased proportionally. Conversely, if the cumulative value of the deviation change rate is negative, it means that the difference between the target attenuation curve and the real-time attenuation curve is narrowing, at which time the length of duration of the sliding evaluation window can be proportionally reduced. In this way, it ensures that the sliding evaluation window can always accurately reflect the attenuation trend of the battery, thereby providing a more precise voltage adaptation reference for the electrothermal therapy device.

The step of correcting the target attenuation curve according to an actual attenuation trend of the battery within the sliding evaluation time window includes:
extracting the real-time voltage, the real-time current, and a capacity attenuation rate of the battery in the sliding evaluation window, and eliminating high-frequency noise by sliding average filtering;
performing a residual analysis on an actual attenuation rate of the sliding evaluation window and a theoretical value of the target attenuation curve to obtain a residual distribution characteristic; and
performing piecewise linear fitting on the target attenuation curve based on the residual distribution characteristic to obtain a correction coefficient, and applying the correction coefficient to a corresponding duration of the target attenuation curve to correct the target attenuation curve, wherein
the target attenuation curve is verified by a validation mechanism after each correction, wherein
when a deviation value between the corrected target attenuation curve and the real-time attenuation curve exceeds a preset verification threshold range under multiple consecutive validation nodes, rolling back to a previous validated correction state, and shortening the length of duration of the sliding evaluation window.

In the embodiment, when correcting the target attenuation curve, it will first extract the real-time voltage, real-time current, and capacity attenuation rate of the battery in the sliding evaluation window to reflect the current attenuation state of the battery. In order to ensure the accuracy of the data, the extracted data will be processed through the sliding average filtering algorithm, so as to eliminate the interference of high-frequency noise and to obtain a smoother attenuation trend curve. Then, the residual analysis is performed on the actual attenuation rate of the sliding evaluation window and a theoretical value of the target attenuation curve, so as to assess the difference between the observed value and the predicted value. The residual distribution characteristic can be obtained by the residual analysis, and the residual distribution characteristic is used to reveal the degree of difference and the distribution pattern between the actual attenuation rate and the target attenuation curve. Next, based on the residual distribution characteristic, the piecewise linear fitting is performed on the target attenuation curve. The correction coefficient of each linear segment can be obtained through the piecewise linear fitting, and the correction coefficient is used to adjust the attenuation rate of the target attenuation curve in the corresponding duration, thereby achieving the correction of the target attenuation curve. After each correction of the target attenuation curve, it needs to be verified through a validation mechanism, thereby ensuring that the corrected curve is more accurate and reliable. The validation mechanism will set multiple verification nodes, and calculate the deviation value between the corrected target attenuation curve and the real-time attenuation curve at each validation node. If the deviation value exceeds the preset verification threshold range for multiple consecutive validation nodes, the correction is considered invalid. At this time, it will roll back to a previous validated correction state, and shorten the length of duration of the sliding evaluation window, which avoids inaccuracy caused by excessive correction and improves the stability and reliability of the correction process.

S5: predicting a remaining usage duration of the battery according to the corrected target attenuation curve, and dynamically adjusting an ablation segment length of an ablation needle according to the remaining usage duration.

As described in step S5, the remaining usage duration of the battery can be predicted based on the corrected target attenuation curve, and then the ablation segment length of the ablation needle can be dynamically adjusted according to the predicted remaining usage duration, so as to adapt to changes of the battery performance and ensure precise control of the ablation process. The step of predicting a remaining usage duration of the battery according to the corrected target attenuation curve, and dynamically adjusting an ablation segment length of an ablation needle according to the remaining usage duration includes:
extracting a correlation function between the capacity attenuation rate and the output voltage based on the corrected target attenuation curve, and calculating a time point of a current capacity reaching a preset failure threshold based on the correlation function, so as to obtain the remaining usage duration of the battery;
establishing a mapping relationship between an ablation segment length and the remaining usage duration, determining the ablation segment where the ablation needle is currently located when the remaining usage duration is smaller than or equal to the preset stability threshold;
reducing the current output when the ablation segment at which the ablation needle is currently located is short-segment ablation; adjusting the long-segment ablation as medium-segment ablation when the current ablation segment is long-segment ablation; shortening an ablation segment length to the short-segment ablation when the remaining usage duration is still insufficient under the medium segment, and continuously reducing the current output; and
maintaining the current ablation segment length and current output unchanged when the remaining usage duration is larger than the preset stability threshold, and monitoring the battery in real time until the ablation process ends.

In the embodiment, when predicting the remaining usage duration of the battery, it will first determine a correlation function (*f*(*v*,*t) = a · v^{b} · e^{-c·t}*, where *f*(*v,t*) represents the capacity attenuation rate; *v* represents the output voltage; t represents the time variable during the ablation process; and *a* , *b* , and *c* represent the fitting parameters) between the capacity attenuation rate and the output voltage based on the corrected target attenuation curve. The correlation function can reflect the dynamic relationship between the capacity attenuation rate and the output voltage of the battery during the attenuation process. Through the application of the correlation function, the time point of the battery reaching the preset failure threshold at the current capacity attenuation rate can be calculated, so as to obtain the remaining usage duration of the battery. After obtaining the remaining usage duration, the current ablation segment at which the ablation needle is located will be determined according to the mapping relationship between the ablation segment length and the remaining usage duration. If the remaining usage duration is less than or equal to the preset stability threshold, it indicates that the battery performance has declined significantly, at which time, it needs to be adjusted according to the current ablation segment where the ablation needle is located, wherein if the ablation needle is currently in the short-segment ablation process, the current output will be further reduced to decrease the burden on the battery; if in the long-segment ablation, it will be adjusted to the medium-segment ablation to balance the ablation effect and the battery performance; and if the remaining usage duration is still insufficient in the medium segment, the ablation segment length will be further shortened to the short-segment ablation, and the current output will be further reduced, so as to ensure precise control of the ablation process. If the remaining usage duration exceeds the preset stability threshold, it indicates that the performance of the battery still remains stable, at which time, the current ablation segment length and the current output can be maintained unchanged, and the battery is monitored in real time. In this way, it can ensure the ablation effect and avoid the unnecessary battery consumption; meanwhile, the real-time monitoring can promptly detect changes of battery performance, thereby providing a corresponding reference for subsequent adjustments.

It should be noted that the ablation needle includes an electrocoagulation function and an ablation function when performing the ablation, wherein
the electrocoagulation function serves for hemostasis and tissue coagulation;
the ablation function serves for an ablation treatment of a target tissue; and
under both the electrocoagulation function and the ablation function, a voltage of each segment of electric heating wire is regulated according to the temperature feedback of temperature measurement components, so as to control the temperature of an entire treatment segment, wherein
the number of the temperature measurement components is larger than the number of segments of electric heating wires, and the temperature measurement components are configured to detect a temperature of a non-target treatment segment.

In the embodiment, when performing the ablation operation, the handheld electrothermal therapy device has dual modes of electrocoagulation function and ablation function, wherein the electrocoagulation function is mainly serves for hemostasis and tissue coagulation, so as to ensure safety during the surgical process, and the ablation function mainly focuses on performing ablation treatment on the target tissue, so as to achieve the expected therapeutic effect. In order to ensure the precision of the temperature control in the therapy process, the voltage of each segment of the electric heating wire will be regulated according to the temperature feedback from the temperature measurement component. It is worth noting that the number of the temperature measurement components is larger than the number of segments of the electric heating wires, which is designed to monitor the temperature of the non-target treatment segment. Through the temperature monitoring of the non-target treatment segment, the condition of overheating or under-temperature can be timely discovered and avoided, thus ensuring the safety and effectiveness of the therapy process.

The illustrative descriptions are as follows.

In the ablation function state,
it is to set the remaining use duration in the current state as T, and to obtain the duration t required for completing ablation according to the size of the tumor volume. If T is larger than t, it is to maintain the current ablation state, and if T is smaller than t, it is to first determine the ablation segment of which the ablation needle is currently located; if the current ablation segment is a short-segment ablation, it is to reduce the current output; if the current ablation segment is a long-segment ablation, it is to adjust the long-segment ablation to the medium-segment ablation; and if the attenuation trend has not been reduced, it is to further adjust the medium-segment ablation to the short-segment ablation and to further reduce the current output.

In addition, it also needs to focus on the attenuation trend of the battery based on the target attenuation curve. If the attenuation trend of the battery continues to accelerate, the ablation strategy needs to be adjusted more frequently, so as to ensure that the ablation operation can be smoothly completed before the battery is exhausted; and at the same time, a warning will be issued to alert the operator to the change of the battery performance and to prepare for the corresponding countermeasures.

In the electrocoagulation function state,
it is to obtain the length of the needle channel where the electrocoagulation and ablation will be performed first, and the length of the needle channel is segmented according to different ablation segment lengths of the needle, so as to obtain the number of times to be ablated (the number of the needle withdrawals) corresponding to different ablation segments; and at the same time, it is to determine the ablation duration and ablation energy corresponding to different ablation segments, so as to select the ablation segment with fewer needle withdrawals and shorter ablation duration to perform the electrocoagulation and ablation for hemostasis. During the process of the ablation for hemostasis, it will also predict the remaining usage duration of the battery.

If the available duration is larger than the ablation duration, it is to maintain the current ablation status unchanged.

If the available duration is smaller than or equal to the ablation duration, the the current ablation segment is determined. If the ablation segment is in the short segment, the current output is reduced; and if the ablation segment is in the medium segment or the long segment, the ablation segment length is gradually reduced in sequence, the power attenuation trend and the usable duration are continuously monitored, and it is determined whether the current output should be further reduced according to the monitoring situation.

The corresponding ablation segment is selected according to the number of needle withdrawals and the ablation duration, the ablation segment is dynamically adjusted in conjunction with the real-time monitoring of the power attenuation trend and the usable duration during the process of use, so that the electrocoagulation and ablation for hemostasis can be completed in the shortest time under the premise of ensuring the safe use of the battery.

It also needs to pay attention to the attenuation trend of the battery herein. If the attenuation trend of the battery suddenly intensifies, it may lead to unstable current during the process of electrocoagulation and ablation for hemostasis, thus affecting the therapeutic effect. Therefore, in the electrocoagulation function state, in addition to adjusting the ablation strategy according to the remaining usage duration and the ablation segment, it also needs to pay attention to the attenuation trend of the battery. Once the abnormal battery attenuation is detected, the ablation operation should be stopped immediately, and the battery should be replaced or other contingency measures should be taken, so as to ensure the safety and effectiveness of the surgery.

Referring to FIG. 2 and FIG. 3, a handheld electrothermal therapy device is provided. The electrothermal therapy device includes:
a handle 1, wherein the handle 1 includes a power supply unit inside, and the power supply unit is configured to provide energy for the ablation and/or electrocoagulation treatment;
a heating head 2, wherein the heating head 2 is provided with a plurality of groups of filling layers 101, temperature measurement units 102, central conductors 103, and electric heating wires 104, so that the heating head performs the electrocoagulation and/or ablation treatment on target tissues of different segment lengths; and
a needle rod 3, wherein one end of the needle rod 3 is connected to the handle 1; one end of the needle rod 3 away from the handle 1 is connected to the heating head 2; and the energy of the power supply unit is transferred to the heating head 2 by conductors inside the needle rod 3, wherein
during the ablation and/or electrocoagulation treatment, the device can obtain a remaining usage duration of the power supply unit according to the attenuation trend of the power supply unit during use, and perform a dynamic adjustment on the ablation segment length according to the remaining usage duration.

Specifically, the handheld electrothermal therapy device is composed of components including the handle 1, the heating head 2, the needle rod 3, and the coaxial puncture needle 4. All parts work in coordination to jointly achieve the multi-functional operation of the electrothermal therapy device. The handle 1, as the main part of the handheld electrothermal therapy device, not only provides the operator a comfortable grip, but also is provided with the power supply unit inside, thereby providing necessary energy support for the entire device. The heating head 2 is a key component of the electrothermal therapy device. It includes multiple groups of complex structures, such as the filling layer 101, the temperature measurement unit 102, the central conductor 103, and the electric heating wire 104, which work together on the target tissue. The filling layer 101 not only has the insulating property to fix the heating wire and prevent short circuit of the electric heating wire, but also has good thermal conductivity, allowing the heat generated by the heating wire 104 to be better transferred out. It can be made of magnesium oxide, and zinc oxide, etc. The temperature measurement unit 102 can be a temperature sensor or other temperature sensing component, such as a thermocouple or thermistor. The material of the central conductor 103 can be a magnesium rod or a silver-plated copper rod. The material of the electric heating wire 104 can be nickel-chromium electric heating alloy, iron-chromium-aluminum electric heating alloy, or copper-nickel alloy, and the non-welded parts thereon are all provided with the magnesium oxide or zinc oxide coating layer. This ensure that, even if the device is operated in the air without load, the electric heating wire will not melt, so as to realize the precise electrocoagulation and/or ablation treatment. The needle rod 3 serves as a bridge connecting the handle 1 and the heating head 2. The energy of the power supply unit is safely and effectively transferred to the heating head 2 by conductors inside, which ensures the smooth progress of the treatment process. The coaxial puncture needle 4 further enhances the puncture and ablation capabilities of the device, which makes the operation more flexible and precise.

In addition, the handheld electrothermal therapy device also includes a start button, a display screen, and a function selection key, wherein the start button is used to control the on/off state of the device; the display screen is used to display various parameters of the device operation; and the function selection key is used to switch different operation modes of the electrothermal therapy device, such as the ablation mode and the electrocoagulation mode, so that an operator can easily switch to the required operation mode according to the actual needs by the function selection key.

It should be noted that the electric heating wires 104 are configured to heat the heating head 2, and a plurality of groups of electric heating wires 104 are provided. One end of each group of electric heating wires 104 is connected to the power supply, and the other end is connected to the central conductor 103. The central conductor 103 is a negative electrode end of all electric heating wires 104, and a loop is formed between the central conductor 103 and the electric heating wires 104. The temperature measurement units 102 are configured to monitor a temperature of the current position, and the number of the temperature measurement units 102 is larger than the number of the electric heating wires 104. The electrocoagulation function serves for hemostasis and coagulation of the tissue.

Specifically, the electric heating wire 104 is provided with the independent heating control capability to achieve precise temperature regulation. Each group of electric heating wires 104 is connected to the power supply unit through an independent power supply line, thereby ensuring that during the electrocoagulation or ablation process, the power can be precisely distributed to different electric heating wires 104 according to actual needs, so as to realize the temperature control in different segments of the heating head 2. The central conductor 103 serves as the common negative electrode of all electric heating wires 104, forming a complete electrical loop with the electric heating wires 104 and ensuring the normal conducting of the current. The temperature measurement units 102 are distributed at different positions of the heating head 2, and are configured for the real-time monitoring of the temperature of each area. Since the number of temperature measurement units 102 is larger than that of electric heating wires 104, it can obtain a more comprehensive understanding of the temperature distribution of the heating head 2, which provides strong support for the control of the ablation temperature. Under the electrocoagulation function, the operator can realize the hemostasis and coagulation operations for the tissue by adjusting the heating power of the electric heating wires 104 and the feedback control of the temperature measurement unit 102, which ensures the safety and effect of the surgical process.

Additionally, the electrocoagulation function serves for hemostasis and coagulation of the tissue, and the ablation function serves for the ablation treatment of the tissue. When using both the electrocoagulation function and the ablation function, the voltage of each segment of electric heating wire 104 is regulated according to the temperature feedback of temperature measurement units 102, so as to control the temperature and the duration of the entire treatment segment, thereby realizing the electrocoagulation function and the ablation function,. This ensures the stability and reliability of the electrothermal therapy device in the process of treatment, and ensures that the electrocoagulation function and the ablation function can be realized smoothly and safely.

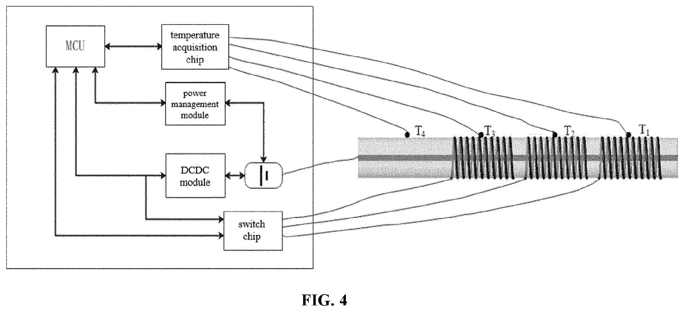

Referring to FIG. 4, during the specific implementation,
the temperature control method is described in the following two examples, but the parameters involved in the examples, such as the set temperature, the number of temperature measurement components, the number of segments of the electric heating wire, the spacing between the heating segments, and the proportionality coefficients, are examples and do not constitute technical limitations.

Reference Example 1. It includes the following setting conditions: selecting only the segment where T1 is located for working; setting the working temperature as T0 = 80°C; and using the electrocoagulation function.

After pressing the start button, the electric heating wire of the segment where T1 is located begins to work. If T1 ≤ T0, it always starts the electric heating wire for heating.

After heating for a while, if 0.9T0 ≤ T1 ≤ 1.1T0, and T2 ≤ 0.6T0, it maintains the present current and continues to work, and it releases the start button until the electrocoagulation is completed.

If T1 > 1.1T0, the MCU controls the switch chip to stop working for 1S, and then it is to restart and appropriately reduce the working current, wherein if T1 can heat up normally, it will continue to use, and if it cannot heat up or the heating is abnormal, it will report an error, stop working, and indicate the device damage.

If 0.9T0≤T1≤1.1T0, but T2 > 0.6T0, it increases the current and reduces the output duty cycle. For example, it switches from continuous working to operating with a moderately increased current at a 70% duty cycle, so as to maintain the temperature of the working segment but to reduce the temperature of the non-working segment. This reduces the unnecessary tissue damage. If the condition of 0.9T0≤T1≤1.1T0 and T2≤0.6T0 can be met, the work continues until the end of the current stage.

After starting, it still shows 0.9T0≤T1≤1.1T0 and T2 > 0.6T0. The MCU controls the switch chip to stop working for 1S, appropriately reduces the working current, and maintains the current duty cycle. Subsequently, the operation is resumed, and the temperature determination method is the same as above. If the condition of 0.9T0≤T1≤1.1T0 and T2≤0.6T0 can be met, the work continues until the end of the current stage.

If it still shows 0.9T0≤T1≤1.1T0 and T2 > 0.6T0, it will continue to stop, appropriately reduce the working current, and maintain the current duty cycle. Subsequently, the operation is resumed. If the condition of 0.9T0≤T1≤1.1T0 and T2≤0.6T0 can be met, the work continues until the end of the current stage. If the condition of 0.9T0≤T1≤1.1T0 and T2≤0.6T0 still cannot be met after three rounds, it will report an error, stop working, and indicate the device damage.

It is to be noted that as used herein, the terms "include", "comprise", or any other variant thereof are intended to cover a non-exclusive inclusion, so that a process, device, item, or method comprising a set of components includes not only those components, but also other component that are not explicitly listed, or component are inherent to this process, device, item, or method. Without further limitation, the fact that a component is defined by the phrase "including a ..." does not exclude the existence of other identical components in the process, device, item, or method including the component.

The foregoing are only optimal embodiments of the present disclosure. It should be noted that for a person of ordinary skill in the art, several improvements and embellishments can be made without departing from the principle of the present disclosure, and such improvements and embellishments should also be considered within the scope of the present disclosure. Structures, devices, and operating methods not specifically described and explained in the present disclosure shall be implemented in accordance with conventional methods in this field, unless otherwise specified or limited.

## Claims

1. An ablation planning method, comprising:
constructing a reference attenuation curve database based on an output voltage, an output current, and a battery capacity in a historical database;
collecting an initial voltage, an initial current, an initial capacity of a battery under a load condition of a constant output voltage, collecting a real-time voltage, a real-time current, and a remaining capacity of the battery according to a preset time interval, and fitting a real-time attenuation curve;
comparing the real-time attenuation curve with reference attenuation curves in a reference attenuation curve database, selecting a reference attenuation curve whose matching degree exceeds a preset matching threshold, summarizing the reference attenuation curve into a candidate attenuation curve dataset, and determining a target attenuation curve adapted to the real-time voltage according to the candidate attenuation curve dataset;
intercepting a sliding evaluation window according to a deviation between the target attenuation curve and the real-time attenuation curve starting from a current ablation node within a remaining usage duration, and correcting the target attenuation curve according to an actual attenuation trend of the battery within the sliding evaluation time window; and
predicting a remaining usage duration of the battery according to the corrected target attenuation curve, and dynamically adjusting an ablation segment length of an ablation needle according to the remaining usage duration.

2. The ablation planning method according to claim 1, wherein the historical database comprises a historical output voltage, a historical output current, and a historical battery capacity under a multi-environmental condition, and the multi-environmental condition comprises temperature, humidity, and load fluctuation; and
when constructing the reference attenuation curve according to the historical output voltage, the historical output current, and the historical battery capacity, the process comprises:
performing a pre-process on the historical output voltage, the historical output current, and the historical battery capacity to remove abnormal data, and performing normalization processing to unify to a same order of magnitude;
performing a cluster analysis on the historical output voltage, the historical output current, and the historical battery capacity according to the multi-environmental condition, so as to obtain a historical data subset under each condition; and
fitting the historical output voltage, the historical output current, and the historical battery capacity in each historical data subset to obtain the reference attenuation curve under each condition, and storing into the reference attenuation curve database.

3. The ablation planning method according to claim 1, wherein the step of collecting a real-time voltage, a real-time current, and a remaining capacity of the battery according to a preset time interval, and fitting a real-time attenuation curve comprises:
dynamically adjusting a sampling interval duration based on a preset ratio according to a current operating temperature and a load fluctuation intensity of the battery;
collecting the real-time voltage and the real-time current of the battery in real time based on the dynamically adjusted sampling interval duration, and recording the remaining capacity of the battery at a corresponding moment; and
fitting the real-time voltage, the real-time current, and the remaining capacity of the battery by using a nonlinear regression method, so as to obtain the real-time attenuation curve of the battery.

4. The ablation planning method according to claim 1, wherein the step of comparing the real-time attenuation curve with reference attenuation curves in a reference attenuation curve database, and selecting a reference attenuation curve whose matching degree exceeds a preset matching threshold comprises:
assessing a morphological consistency between the real-time attenuation curve and the reference attenuation curve through a waveform comparison;
performing a time axis calibration on the curves under different collection frequencies, and eliminating a sampling interval error by using difference compensation, so as to obtain the calibrated real-time attenuation curve and reference attenuation curve;
establishing a two-stage screening mechanism, wherein a reference attenuation curve with a morphological consistency higher than a preliminary threshold is retained under a first-stage screening mechanism, and an abnormal matching curve in the remaining reference attenuation curves is eliminated by a statistical validation under a second-stage screening mechanism; and
taking the remaining reference attenuation curves after screened by the two-stage screening mechanism as the candidate attenuation curve, and summarizing the candidate attenuation curve into the candidate attenuation curve dataset.

5. The ablation planning method according to claim 1, wherein the step of determining a target attenuation curve adapted to the real-time voltage according to the candidate attenuation curve dataset comprises:
performing time synchronization processing on the candidate attenuation curve and the real-time attenuation curve, calculating a time series similarity between the candidate attenuation curve and the real-time attenuation curve, and screening a candidate attenuation curve exceeding a time series similarity threshold;
acquiring a current environmental parameter, and performing a multi-level filtering operation according to a different value between a historical temperature and a current temperature, a load fluctuation amplitude, and an environmental humidity; and
performing a trend validation on the candidate attenuation curve passing the multi-level filtering operation, and selecting the candidate attenuation curve with a highest matching degree of the attenuation trend to the real-time attenuation trend as the target attenuation curve.

6. The ablation planning method according to claim 1, wherein the step of intercepting a sliding evaluation window according to a deviation between the target attenuation curve and real-time attenuation curve comprises:
tracing back a sample duration on the basis of a current node, and setting multiple sampling nodes within the sample duration;
obtaining a cumulative deviation value between the target attenuation curve and the real-time attenuation curve at each sampling node, and triggering a window interception mechanism when the cumulative deviation value exceeds a preset deviation threshold;
calculating a deviation change rate of the cumulative deviation value at each sampling node, and adaptively adjusting a length of duration of the sliding evaluation window according to the deviation change rate;
extending the length of duration of the sliding evaluation window to twice a length of baseline when deviation change rates of N consecutive sampling nodes exceed a preset deviation change rate threshold;
shortening the length of duration of the sliding evaluation window to half of the length of baseline when the deviation change rates of N consecutive sampling nodes are lower than a preset deviation stability threshold;
adjusting the length of duration of the sliding evaluation window according to a cumulative value of the deviation change rates when the deviation change rates at N sampling nodes exceed the preset deviation change rate threshold and are lower than the preset deviation stability threshold simultaneously; and
proportionally increasing the length of duration of the sliding evaluation window when the cumulative value of the deviation change rates is positive, and proportionally reducing the length of duration of the sliding evaluation window when the cumulative value of the deviation change rates is negative.

7. The ablation planning method according to claim 1, wherein the step of correcting the target attenuation curve according to an actual attenuation trend of the battery within the sliding evaluation time window comprises:
extracting the real-time voltage, the real-time current, and a capacity attenuation rate of the battery in the sliding evaluation window, and eliminating high-frequency noise by sliding average filtering;
performing a residual analysis on an actual attenuation rate of the sliding evaluation window and a theoretical value of the target attenuation curve to obtain a residual distribution characteristic; and
performing piecewise linear fitting on the target attenuation curve based on the residual distribution characteristic to obtain a correction coefficient, and applying the correction coefficient to a corresponding duration of the target attenuation curve to correct the target attenuation curve, wherein
the target attenuation curve is verified by a validation mechanism after each correction; and
when a deviation value between the corrected target attenuation curve and the real-time attenuation curve exceeds a preset verification threshold range under multiple consecutive validation nodes, rolling back to a previous validated correction state, and shortening the length of duration of the sliding evaluation window.

8. The ablation planning method according to claim 1, wherein the step of predicting a remaining usage duration of the battery according to the corrected target attenuation curve, and dynamically adjusting an ablation segment length of an ablation needle according to the remaining usage duration comprises:
extracting a correlation function between the capacity attenuation rate and the output voltage based on the corrected target attenuation curve, and calculating a time point of a current capacity reaching a preset failure threshold based on the correlation function, so as to obtain the remaining usage duration of the battery;
establishing a mapping relationship between an ablation segment length and the remaining usage duration, determining the ablation segment where the ablation needle is currently located when the remaining usage duration is smaller than or equal to the preset stability threshold;
reducing the current output when the ablation segment where the ablation needle is currently located is short-segment ablation; adjusting the long-segment ablation as medium-segment ablation when the current ablation segment is long-segment ablation; shortening an ablation segment length to the short-segment ablation when the remaining usage duration is still insufficient under the medium segment, and continuously reducing the current output; and
maintaining the current ablation segment length and current output unchanged when the remaining usage duration is larger than the preset stability threshold, and monitoring the battery in real time until the ablation process ends.

9. The ablation planning method according to claim 8, wherein the ablation needle comprises an electrocoagulation function and an ablation function when performing the ablation;
the electrocoagulation function serves for hemostasis and tissue coagulation;
the ablation function serves for an ablation treatment of a target tissue; and
when using both the electrocoagulation function and the ablation function, a voltage of each segment of electric heating wire is regulated according to temperature feedback of temperature measurement components, so as to control a temperature of an entire treatment segment; and
the number of the temperature measurement components is larger than the number of segments of electric heating wires, and the temperature measurement components are configured to detect a temperature of a non-target treatment segment.

10. A handheld electrothermal therapy device, applicable for the ablation planning method according to any one of claims 1 to 9, wherein the electrothermal therapy device comprises:
a handle (1), wherein the handle (1) comprises a power supply unit inside, and the power supply unit is configured to provide energy for the ablation and/or electrocoagulation treatment;
a heating head (2), wherein the heating head (2) is provided with a plurality of groups of filling layers (101), temperature measurement units (102), central conductors (103), and electric heating wires (104), so that the heating head (2) performs the electrocoagulation and/or ablation treatment on target tissues of different segment lengths; and
a needle rod (3), wherein one end of the needle rod (3) is connected to the handle (1); one end of the needle rod (3) away from the handle (1) is connected to the heating head (2); and the energy of the power supply unit is transferred to the heating head (2) by conductors inside the needle rod (3); and
during the ablation and/or electrocoagulation treatment, the device is able to obtain a remaining usage duration of the power supply unit according to the attenuation trend of the power supply unit during use, and perform a dynamic adjustment on the ablation segment length according to the remaining usage duration.

11. The handheld electrothermal therapy device according to claim 10, wherein a coaxial puncture needle (4) for use in conjunction with the electrothermal therapy device is provided when the electrothermal therapy device is in use, and the electrothermal therapy device achieves the electrocoagulation and/or ablation treatment through a channel established by the coaxial puncture needle.

12. The handheld electrothermal therapy device according to claim 10, wherein the electric heating wires (104) are configured to heat the heating head (2), a plurality of groups of electric heating wires (104) are provided, one end of each group of electric heating wires (104) is connected to the power supply, and the other end is connected to the central conductor (103).

13. The handheld electrothermal therapy device according to claim 11, wherein the central conductor (103) is a negative electrode end of all electric heating wires (104), and a loop is formed between the central conductor (103) and the electric heating wires (104).

14. The handheld electrothermal therapy device according to claim 10, wherein each of the temperature measurement units (102) is configured to monitor a temperature of a current position, and the number of the temperature measurement units (102) is larger than the number of the electric heating wires (104).

15. The handheld electrothermal therapy device according to claim 10, wherein the electrocoagulation function serves for hemostasis and coagulation of the tissue;
the ablation function serves for the ablation treatment of the tissue; and
when using both the electrocoagulation function and the ablation function, the voltage of each segment of electric heating wire (104) is regulated according to the temperature feedback of temperature measurement units (102), so as to control the temperature and the duration of the entire treatment segment, thereby realizing the electrocoagulation function and the ablation function.
